# EUROPEAN PATENT SPECIFICATION

(11) **EP 4 245 271 B1**
(45) Date of publication and mention of the grant of the patent: **09.07.2025**
(21) Application number: 23161957.8
(22) Date of filing: 15.03.2023
(51) Int. Cl.: A61F 2/66

(54) **INTEGRATED PROSTHETIC FOOT WITH TRANSVERSE ARCH**
INTEGRIERTER FUSSPROTHESE MIT QUERBOGEN
PROTHÈSE DE PIED INTÉGRÉE AVEC ARC TRANSVERSAL

(30) Priority: 18.03.2022 CN 202210270474
(43) Date of publication of application: 20.09.2023
(73) Proprietor: Jilin University, Changchun, Jilin Province 130012 (CN)
(72) Inventor: REN, Lei, Changchun, 130012 (CN); WANG, Kunyang, Changchun, 130012 (CN); XU, Xiaohan, Changchun, 130012 (CN); QIAN, Zhihui, Changchun, 130012 (CN); XUE, Yifeng, Changchun, 130012 (CN); LU, Xuewei, Changchun, 130012 (CN); LIANG, Wei, Changchun, 130012 (CN); XIU, Haohua, Changchun, 130012 (CN); REN, Luquan, Changchun, 130012 (CN)
(74) Representative: Neugebauer, Jürgen

(56) References cited:
- CN-A- 112 206 084
- CN-A- 113 332 010
- CN-A- 115 501 014
- US-A1- 2006 069 450

## Description

### Technical Field

The present invention relates to the technical field of manufacturing of artificial limb, and in particular to an integrated prosthetic foot with transverse arch.

### Background

The disabled people totaled 85.01 million in China by the end of 2010, including 24.72 million physically disabled ones. Amputation not only brings burdens to patients and their families, but also greatly affects their psychological state and interpersonal relationship. Prostheses can be used to replace limbs to a certain extent in terms of motor function, and are the best means to help amputees improve their mobility and fit into social life at present. A prosthetic foot substitutes for human foot and ankle system, and its performance directly affects the overall function of the prosthesis as a main component of the later. Previously, it was mainly designed based on a human foot longitudinal arch feature in order to increase its energy storage capacity and overall elasticity, and reduce the impact on the sole upon landing. In the prior art, carbon fiber composite materials are usually used to manufacture the prosthetic foot, and make, on accounts of their directionality, the carbon fiber prosthetic foot have higher stiffness in the direction of longitudinal arc, which is contrary in the direction of transverse arch. And, a transversely flat and even-thick prosthetic foot has poor dynamic response to different gaits during support, causing impact force to be transmitted to stumps, and further affecting comfort and stability of the prosthesis. Relevant researches show that a human foot transverse arch feature can effectively improve the overall stiffness of the sole, and play a vital role in different motion states. To this end, it is necessary to design a new prosthetic foot having a transverse arch combining a curvature change feature of the human foot transverse arc, which can improve the overall stiffness of the prosthetic foot, effectively reduce the weight of the sole, and effectively improve the stability and comfort of the prosthesis under different road conditions.

US20060069450A1 discloses a foot prosthesis having improved rollover and stability. The prosthesis includes a first plate and a mounting block having a mounting portion configured to be coupled to a user of the prosthesis, an attachment portion and a gap portion, with the mounting block attached to the first plate at the attachment portion with a gap between the mounting block gap portion and the first plate. Also included is a resilient element positioned at least partially within the gap, the resilient element configured generally to dissipate stress in the first plate and control deflection between the first plate and the mounting block. The mounting block may be substantially rigid with the area of the first plate attached to the mounting block becoming substantially rigid while the remainder of the first plate is at least partially flexible. The resilient element may be interchangeable to adjust performance of the prosthesis.

CN113332010A discloses a separated prosthetic foot with a transverse arch, which belongs to the technical field of manufacturing of artificial limbs. The characteristics of the transverse arch of the human foot are combined into the previous transverse-straight carbon fiber foot design to improve the stiffness of the prosthetic foot and reduce the weight of the prosthetic foot. A forefoot part employs a separated curved plate design, and two simple curved plates with different curvatures are used for simulating a complex curved surface of the transverse arch of the human, such that the manufacturing cost is reduced, and the various parts of the sole are non-uniform in stiffness distribution: the outer side of the foot is a single-layer forefoot plate with a smaller curvature and minor stiffness; and the inner side of the foot is a double-layer stacked forefoot plate, and the lower layer plate has a large radius in curvature and stiffness.

CN112206084A discloses a prosthetic foot. The prosthetic foot has a gradient rigidity change of a three-dimensional curvature feature of an arch of a human body and belongs to a technical field of artificial prosthesis manufacturing. The prosthetic foot includes a forefoot carbon fiber curved plate, a heel carbon fiber curved plate and a connection assembly, a three-dimensional curved surface shape of the prosthetic foot is designed in combination with the three-dimensional curvature features of a longitudinal arch and a transverse arch of a human foot, and the prosthetic foot with gradient rigidity distribution is provided. Compared with a carbon fiberfootstraight in a transverse direction, on one hand, weight of the prosthetic foot is reduced while overall rigidity of the prosthetic foot is improved, so as to achieve better supporting and propelling effects and lighter and more comfortable use experience; and on the other hand, the gradient rigidity change, with larger rigidity of a rear part, smaller rigidity of a front part, larger rigidity of inner partial toes and smaller rigidity of outer partial toes, of the prosthetic foot dynamically meets different requirements of different road conditions and different walking stages on the rigidity of different parts of the prosthetic foot, such that the response of the prosthetic foot to movement is more intelligent, timely and reliable.

### Summary

An objective of the present invention is to provide an integrated prosthetic foot combining a human foot transverse arch feature and a changing rule of a plantar pressure in a gait cycle aiming at the shortcomings of an existing carbon fiber prosthetic foot. A three-dimensional curvature feature of a human foot arch is combined into design of a transversely flat carbon fiber prosthetic foot, thereby satisfying rigidity requirements from the prosthetic foot, greatly reducing a weight of the prosthetic foot, and providing higher wearing comfort for a patient; the prosthetic foot adopts integrated curved plate design, and a forefoot and a heel are designed into a curved plate having functions of the forefoot and the heel, and has features of a longitudinal arch and a transverse arch from the heel to the forefoot, thereby greatly simplifying disassembly and assembly processes and reducing manufacturing cost.

An integrated prosthetic foot with transverse arch of the present invention includes a sole curved plate A, a adapting piece B, a first bolt group 1, a second bolt group 2, a third bolt group 3 and a fourth bolt group 4, wherein the sole curved plate A is a carbon fiber composite plate, and includes a rear plate 5a, a rear transition plate 5b, a middle arc plate 5c, a middle transition plate 5d and a front flat plate 5e, the rear plate 5a, the rear transition plate 5b and the middle arc plate 5c, the middle transition plate 5d and the front flat plate 5e are smoothly connected into a whole unit in sequence from back to front.

The adapting piece B is made of aluminum alloy, and includes a square frustum 6a, a platform 6b and a main body 6c, the square frustum 6a is fixedly connected to a rear portion of the main body 6c by means of the platform 6b; the rear flat plate 5a and the front flat plate 5e are broken lines, the broken lines are formed by connecting two straight lines with a height difference of 2mm by means of arc transition; the rear transition plate 5b and the middle transition plate 5d each smoothly transition from an upward convex arc shape to a straight shape, with an arc radius of 20mm-25mm, a central angle of 80°-85°, and a curvature going from large to small; and the middle arc plate 5c has a transverse curve designed with reference to a human foot transverse arch curve, and a front end and a back end are connected by means of a smooth arc transition.

The sole curved plate A is wholly defined by a longitudinal sole curve C, an outer longitudinal curve D and an inner longitudinal curve E in a front and back direction, the longitudinal sole curve C includes a first longitudinal curve 5j, a second longitudinal curve 5k and a third longitudinal line 5l; the first longitudinal 5j is a downward concave arc with an arc radius of 60mm-70mm and a central angle of 43°-45°, the second longitudinal curve 5k is an upward concave arc with an arc radius of 50mm-60mm and a central angle of 74°-76°, and the first longitudinal curve 5j and the second longitudinal curve 5k are connected in sequence and smoothly in a tangential manner from back to front; and the third longitudinal curve 5l is a downward concave arc with an arc radius of 100mm-110mm and a central angle of 40°-42°, and the second longitudinal curve 5k and the third longitudinal curve 5l are connected in sequence and smoothly in a tangential manner from back to front.

Shapes of the outer longitudinal curve D and the inner longitudinal curve E are drawn according to a basic shape of a human longitudinal arch and have a greater inner curvature and a smaller outer curvature.

A mathematical expression of the outer longitudinal curve D is: y = -0.0015x³+0.3313x²-3.5641x.

A mathematical expression of the inner longitudinal curve E is: y = -0.0025x³+0.5492x²-9.6027x.

The adapting piece B and the sole curved plate A are arranged vertically, and are fixedly connected by the first bolt group 1, the second bolt group 2, the third bolt group 3 and the fourth bolt group 4.

A bottom surface of the main body 6c of the adapting piece B has the same size as arc surfaces of the rear transition plate 5b, the middle arc plate 5c and the middle transition plate 5d of the sole curved plate A.

A middle potion of the main body 6c is provided with three elliptical holes of a weight-reducing hole group 6g, a short axis of an ellipse is 2mm-8mm, and a long axis of the ellipse is 9mm-30mm.

The bottom surface of the main body 6c is a curved surface designed per the human foot transverse arch curve, and fits an upper surface of the sole curved plate A.

The rear transition plate 5b of the sole curved plate A is provided with a first rear hole 5i, the middle arc plate 5c is provided with a first middle hole 5g and a second middle hole 5h, and the middle transition plate 5d is provided with a first front hole 5f; and a bottom of the main body 6c of the adapting piece B is provided with a second front hole 6d, a third middle hole 6e, a fourth middle hole 6f and a second rear hole 6h.

The second rear hole 6h, the third middle hole 6e, the fourth middle hole 6f and the second front hole 6d in sequence correspond vertically to the first rear hole 5i, the second middle hole 5h, the first middle hole 5g and the first front hole 5f respectively.

In a complete gait cycle, a plantar pressure center starts at the heel touching ground, gradually moves forward from the heel to outside of the forefoot, and then moves to inside of the forefoot during the human foot touches the ground completely. Plantar pressures on a human foot are not uniform in different stages, so stiffness required for different portions of the sole to bear the pressures is also different. Therefore, sole longitudinal arch curves of inside and outside of the prosthetic foot of the present invention are different, that is, the sole has a smaller curvature on the outer longitudinal curve D, and a larger deformation amount when being stressed, such that impact force generated when the sole touches the ground is absorbed while the sole may conveniently adapt to various road conditions, buffering and energy storing are achieved, and comfort of a wearer during walking is improved; and the sole has a larger curvature on the inner longitudinal curve E, so as to provide a greater rigidity for the prosthetic foot and a better support and propulsion during walking. In the gait cycle, when the sole touches the ground, a center of gravity of a human body first falls on the back flat plate 5a of the sole curved plate A, and then gradually shifts from the back flat plate 5a to the outer longitudinal curve D of the front flat plate 5e; and then the impact force is transferred from the outer longitudinal curve D of the front plate 5e to the inner longitudinal curve E of the front plate 5e.

The present invention has the following beneficial effects:
1. The present invention combines the human transverse arch feature and the human longitudinal arch feature into the design of the prosthetic foot, and improves the traditional transversely flat carbon fiber plate into the transverse curved carbon fiber plate having a transverse arch feature, which may provide the rigidity required by the patient during walking under various road conditions and effectively reduce the weight of the prosthetic foot; the sole adopts integrated curved plate design, a simple carbon fiber curved plate is used as the main body of the prosthetic foot, and a forefoot and a heel of a conventional prosthetic foot are combined to form one curved plate, such that the prosthetic foot has the functions of the forefoot and the heel, thereby greatly simplifying disassembly and assembly difficulty and reducing the manufacturing cost and difficulty.
2. The sole of the prosthetic foot is designed with reference to the change rule of the plantar pressure center, rigidity distribution over various portions is made nonuniform by making the curvature of the inner and outer longitudinal curves of the sole different, such that the inner curvature of the sole is larger and the rigidity is larger; the outer curvature of the sole is smaller, and the rigidity is smaller; and the design of different curvature causes the prosthetic foot to satisfy the requirements of stiffness and comfort at different walking stages in the gait cycle.

### Brief Description of the Drawings

Fig. 1 shows a first axonometric view (in a front right direction) of an integrated transverse arch prosthetic foot;
Fig. 2 shows a second axonometric view (in a rear right direction) of the integrated transverse arch prosthetic foot;
Fig. 3 shows a third axonometric view (in a bottom right direction) of the integrated transverse arch prosthetic foot;
Fig. 4 shows an explosive view (in a front right direction) of the integrated transverse arch prosthetic foot;
Fig. 5 shows a first axonometric view (in a front right direction) of segmented cross sections of a sole curved plate A;
Fig. 6 shows a second axonometric view (in a bottom right direction) of segmented cross sections of the sole curved plate A;
Fig. 7 shows a right side view of a longitudinal sole curve C;
Fig. 8 shows an axonometric view (in a front right direction) of an outer longitudinal curve D and an inner longitudinal curve E;
Fig. 9 shows a first axonometric view (in a front right direction) of a adapting piece B; and
Fig. 10 shows a second axonometric view (in a rear right direction) of the adapting piece B.

In the figures: A. a sole curved plate; B. a adapting piece; C. a longitudinal sole curve; D. an outer longitudinal curve; E. an inner longitudinal curve; 1. a first bolt group; 2. a second bolt group; 3. a third bolt group; 4. a fourth bolt group; 5a. a rear flat plate; 5b. a rear transition plate; 5c. a middle arc plate; 5d. a middle transition plate; 5e. a front flat plate; 5f. a first front hole; 5g. a second middle hole; 5h. a first middle hole; 5i. a first rear hole; 5j. a first longitudinal curve; 5k. a second longitudinal curve; 5l. a third longitudinal curve; 6a. a square frustum; 6b. a platform; 6c. a main body; 6d. a second front hole; 6e. a third middle hole; 6f. a fourth middle hole; 6g. a weight-reducing hole group; 6h. a second rear hole.

### Detailed Description of the Embodiments

The present invention will be described below with reference to accompanying drawings.

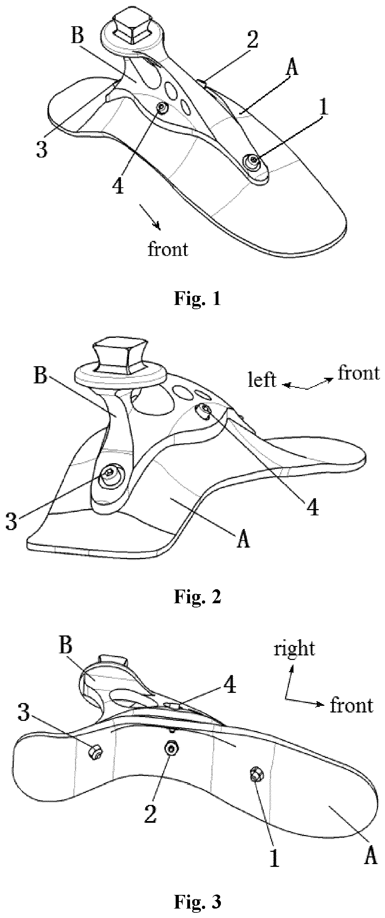

As shown in Figs. 1-4, the present invention includes a sole curved plate A, a adapting piece B, a first bolt group 1, a second bolt group 2, a third bolt group 3 and a fourth bolt group 4. A bottom surface of a main body 6c of the adapting piece B has the same size as arc surfaces of a rear transition plate 5b, a middle arc plate 5c and a middle transition plate 5d of the sole curved plate A. A second rear hole 6h, a third middle hole 6e, a fourth middle hole 6f and a second front hole 6d in sequence correspond vertically to a first rear hole 5i, a second middle hole 5h, a first middle hole 5g and a first front hole 5f respectively. The adapting piece B and the sole curved plate A are arranged vertically, and are fixedly connected by the first bolt group 1, the second bolt group 2, the third bolt group 3 and the fourth bolt group 4.

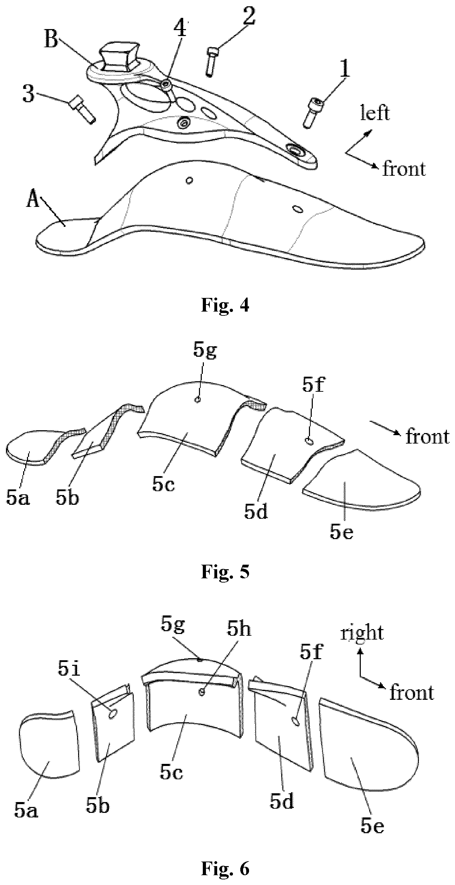

As shown in Figs. 5-6, the sole curved plate A is a carbon fiber composite plate, and includes a rear plate 5a, the rear transition plate 5b, the middle arc plate 5c, the middle transition plate 5d and a front flat plate 5e, the rear plate 5a, the rear transition plate 5b and the middle arc plate 5c, the middle transition plate 5d and the front flat plate 5e are smoothly connected into a whole unit in sequence from back to front. The rear flat plate 5a and the front flat plate 5e are broken lines, the broken lines are formed by connecting two straight lines with a height difference of 2mm by means of arc transition. The rear transition plate 5b and the middle transition plate 5d each smoothly transition from an upward convex arc shape to a straight shape, with an arc radius of 20mm-25mm, a central angle of 80°-85°, and a curvature going from large to small. And the middle arc plate 5c has a transverse curve designed with reference to a human foot transverse arch curve, and a front end and a back end are connected by means of a smooth arc transition.

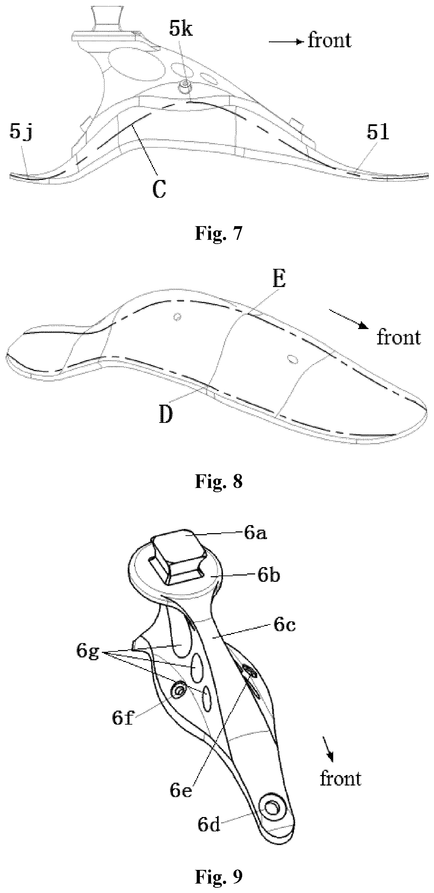

As shown in Figs. 7-8, the sole curved plate A is wholly defined by a longitudinal sole curve C, an outer longitudinal curve D and an inner longitudinal curve E in a front and back direction, the longitudinal sole curve C includes a first longitudinal curve 5j, a second longitudinal curve 5k and a third longitudinal curve 5l. The first longitudinal 5j is a downward concave arc having an arc radius of 60mm-70mm and a central angle of 43°-45°, the second longitudinal curve 5k is an upward concave arc having an arc radius of 50mm-60mm and a central angle of 74°-76°, and the first longitudinal curve 5j and the second longitudinal curve 5k are connected in sequence and smoothly in a tangential manner from back to front. And the third longitudinal curve 5l is a downward concave arc having an arc radius of 100mm-110mm and a central angle of 40°-42°, and the second longitudinal curve 5k and the third longitudinal curve 5l are connected in sequence and smoothly in a tangential manner from back to front.

Shapes of the outer longitudinal curve D and the inner longitudinal curve E are drawn according to a basic shape of a human longitudinal arch and have a greater inner curvature and a smaller outer curvature.

A mathematical expression of the outer longitudinal curve D is: y = -0.0015x³+0.3313x²-3.5641x.

A mathematical expression of the inner longitudinal curve E is: y = -0.0025x³+0.5492x²-9.6027x.

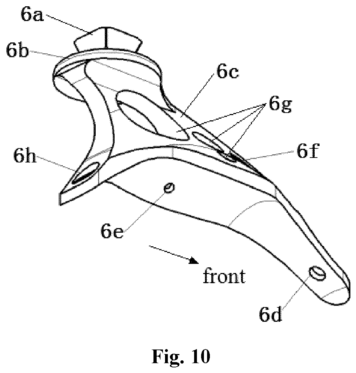

As shown in Figs. 9-10, the adapting piece B is made of aluminum alloy, and includes a square frustum 6a, a platform 6b and a main body 6c, the square frustum 6a is fixedly connected to a rear portion of the main body 6c by means of the platform 6b. A bottom of the main body 6c of the adapting piece B is provided with a second front hole 6d, a third middle hole 6e, a fourth middle hole 6f and a second rear hole 6h. A middle potion of the main body 6c is provided with three elliptical holes of a weight-reducing hole group 6g, a short axis of an ellipse is 2mm-8mm, and a long axis of the ellipse is 9mm-30mm. And the bottom surface of the main body 6c is a curved surface designed per the human foot transverse arch curve, and fits an upper surface of the sole curved plate A.

## Claims

1. An integrated prosthetic foot with transverse arch, comprising a sole curved plate (A), an adapting piece (B), a first bolt group (1), a second bolt group (2), a third bolt group (3) and a fourth bolt group (4), wherein the sole curved plate (A) comprises a rear flat plate (5a), a rear transition plate (5b), a middle arc plate (5c), a middle transition plate (5d) and a front flat plate (5e), the rear flat plate (5a), the rear transition plate (5b), the middle arc plate (5c), the middle transition plate (5d) and the front flat plate (5e) are smoothly connected into a whole unit in sequence from back to front; the adapting piece (B) comprises a square frustum (6a), a platform (6b) and a main body (6c), the square frustum (6a) is fixedly connected to a rear portion of the main body (6c) by means of the platform (6b); the rear flat plate (5a) and the front flat plate (5e) are broken lines, the broken lines are formed by connecting two straight lines with a height difference of 2mm by means of arc transition; the rear transition plate (5b) and the middle transition plate (5d) each smoothly transition from an upward convex arc shape to a straight shape, with an arc radius of 20mm-25mm, a central angle of 80°-85°, and a curvature going from large to small; the middle arc plate (5c) has a transverse curve designed with reference to a human foot transverse arch curve, and a front end and a back end are connected by means of a smooth arc transition; the sole curved plate (A) is wholly defined by a longitudinal sole curve (C), an outer longitudinal curve (D) and an inner longitudinal curve (E) in a front and back direction, the longitudinal sole curve (C) comprises a first longitudinal curve (5j), a second longitudinal curve (5k) and a third longitudinal curve (5l); the first longitudinal curve (5j) is a downward concave arc with an arc radius of 60mm-70mm and a central angle of 43°-45°, the second longitudinal curve (5k) is an upward concave arc with an arc radius of 50mm-60mm and a central angle of 74°-76°, and the first longitudinal curve (5j) and the second longitudinal curve (5k) are connected in sequence and smoothly in a tangential manner from back to front; the third longitudinal curve (5l) is a downward concave arc with an arc radius of 100mm-110mm and a central angle of 40°-42°, and the second longitudinal curve (5k) and the third longitudinal curve (5l) are connected in sequence and smoothly in a tangential manner from back to front; shapes of the outer longitudinal curve (D) and the inner longitudinal curve (E) are drawn according to a basic shape of a human longitudinal arch and have a greater inner curvature and a smaller outer curvature;
a mathematical expression of the outer longitudinal curve (D) is: y = -0.0015x³+0.3313x²-3.5641x;
a mathematical expression of the inner longitudinal curve (E) is: y = -0.0025x³+0.5492x²-9.6027x; and
the adapting piece (B) and the sole curved plate (A) are arranged vertically, and are fixedly connected by the first bolt group (1), the second bolt group (2), the third bolt group (3) and the fourth bolt group (4); a bottom surface of the main body (6c) of the adapting piece (B) has the same size as arc surfaces of the rear transition plate (5b), the middle arc plate (5c) and the middle transition plate (5d) of the sole curved plate (A); a middle potion of the main body (6c) is provided with three elliptical holes of a weight-reducing hole group (6g), a short axis of an ellipse is 2mm-8mm, and a long axis of the ellipse is 9mm-30mm; and the bottom surface of the main body (6c) is a curved surface designed per the human foot transverse arch curve, and fits an upper surface of the sole curved plate (A).

2. The integrated prosthetic foot with transverse arch as claimed in claim 1, wherein the rear transition plate (5b) of the sole curved plate (A) is provided with a first rear hole (5i), the middle arc plate (5c) is provided with a first middle hole (5g) and a second middle hole (5h), and the middle transition plate (5d) is provided with a first front hole (5f); a bottom of the main body (6c) of the adapting piece (B) is provided with a second front hole (6d), a third middle hole (6e), a fourth middle hole (6f) and a second rear hole (6h); and the second rear hole (6h), the third middle hole (6e), the fourth middle hole (6f) and the second front hole (6d) in sequence correspond vertically to the first rear hole (5i), the second middle hole (5h), the first middle hole (5g) and the first front hole (5f) respectively.

3. The integrated prosthetic foot with transverse arch as claimed in claim 1, wherein the sole curved plate (A) is a carbon fiber composite plate, and the adapting piece (B) is made of aluminum alloy.

## Patentansprüche

1. Integrierte Fußprothese mit Querbogen, die eine gekrümmte Sohlenplatte, (A), ein Anpassungsstück (B), eine erste Schraubengruppe (1), eine zweite Schraubengruppe (2), eine dritte Schraubengruppe (3) und eine vierte Schraubengruppe (4) umfasst, wobei die gekrümmte Sohlenplatte (A) eine hintere flache Platte (5a), eine hintere Übergangsplatte (5b), eine mittlere Bogenplatte (5c), eine mittlere Übergangsplatte (5d) und eine vordere flache Platte (5e) umfasst, die hintere flache Platte (5a), die hintere Übergangsplatte (5b), die mittlere Bogenplatte (5c), die mittlere Übergangsplatte (5d) und die vordere flache Platte (5e) der Reihe nach von hinten nach vorn fugenlos als eine komplette Einheit verbunden sind; das Anpassungsteil (B) einen quadratischen Stumpf (6a), einen Sockel (6b) und einen Hauptteil (6c) umfasst, der quadratische Stumpf (6a) fest mit einem hinteren Abschnitt des Hauptteils (6c) über den Sockel (6b) verbunden ist; die hintere flache Platte (5a) und die vordere flache Platte (5e) unterbrochene Linien sind, die unterbrochenen Linien durch Verbinden von zwei geraden Linien mit einem Höhenunterschied von 2 mm über einen Bogenübergang gebildet sind; die hintere Übergangsplatte (5b) und die mittlere Übergangsplatte (5d) jeweils sanft von einer nach oben konvexen Bogenform in eine gerade Form übergehen, mit einem Bogenradius von 20mm-25mm, einem Mittelpunktswinkel von 80°-85° und einer Wölbung von stark zu schwach; die mittlere Bogenplatte (5c) eine quer verlaufende Krümmung aufweist, die unter Bezug auf die Krümmung eines Querbogens eines menschlichen Fußes ausgestaltet ist, und ein vorderes Ende und ein hinteres Ende über einen sanften Bogenübergang verbunden sind; die gekrümmte Sohlenplatte (A) in einer Vorwärts-Rückwärts-Richtung vollständig durch eine Sohlenlängswölbung (C), eine äußere Längswölbung (D) und eine innere Längswölbung (E) definiert ist, die Sohlenlängswölbung (C) eine erste Längswölbung (5j), eine zweite Längswölbung (5k) und eine dritte Längswölbung (51) umfasst; die erste Längswölbung (5j) ein nach unten konkaver Bogen mit einem Bogenradius von 60 mm-70 mm und einem Mittelpunktswinkel von 43°-45° ist, die zweite Längswölbung (5k) ein nach oben konkaver Bogen mit einem Bogenradius von 50mm-60mm und einem Mittelpunktswinkel von 74°-76° ist und die erste Längswölbung (5j) und die zweite Längswölbung (5k) nacheinander und fugenlos tangential von hinten nach vorn verbunden sind; die dritte Längswölbung (51) ein nach unten konkaver Bogen mit einem Bogenradius von 100mm-110mm und einem Mittelpunktswinkel von 40°-42° ist und die zweite Längswölbung (5k) und die dritte Längswölbung (51) nacheinander und fugenlos tangential von hinten nach vorn verbunden sind; Formen der äußeren Längswölbung (D) und der inneren Längswölbung (E) einer Grundform eines Längsbogens eines Menschen entsprechend gestaltet sind und eine stärkere Innenwölbung und eine schwächere Außenwölbung aufweisen;
ein mathematischer Ausdruck für die äußere Längswölbung (D) folgendermaßen lautet: y = -0,0015x³+0,3313x²-3,5641x;
ein mathematischer Ausdruck für die innere Längswölbung (E) folgendermaßen lautet: y = -0, 0025x³+0,5492x²-9,6027x; und
das Anpassungsstück (B) und die gekrümmte Sohlenplatte (A) vertikal angeordnet sind und über die erste Schraubengruppe (1), die zweite Schraubengruppe (2), die dritte Schraubengruppe (3) und die vierte Schraubengruppe (4) fest verbunden sind; eine untere Fläche des Hauptteils (6c) des Anpassungsstücks (B) genau so groß ist wie Bogenflächen der hinteren Übergangsplatte (5b), der mittleren Bogenplatte (5c) und der mittleren Übergangsplatte (5d) der gekrümmten Sohlenplatte (A); ein mittlerer Abschnitt des Hauptteils (6c) mit drei elliptischen Löchern einer gewichtsverringernden Lochgruppe (6g) versehen ist, eine kurze Achse einer Ellipse 2mm-8mm misst und eine lange Achse der Ellipse 9mm-30mm misst; und die untere Fläche des Hauptteils (6c) eine gekrümmte Fläche ist, die der Krümmung des Querbogens eines menschlichen Fußes entsprechend ausgestaltet ist und einer oberen Fläche der gekrümmten Sohlenplatte (A) entspricht.

2. Integrierte Fußprothese mit Querbogen nach Anspruch 1, wobei die hintere Übergangsplatte (5b) der gekrümmten Sohlenplatte (A) mit einem ersten hinteren Loch (5i) versehen ist, die mittlere Bogenplatte (5c) mit einem ersten mittleren Loch (5g) und einem zweiten mittleren Loch (5h) versehen ist, und die mittlere Übergangsplatte (5d) mit einem ersten vorderen Loch (5f) versehen ist; ein Boden des Hauptteils (6c) des Anpassungsstücks (B) mit einem zweiten vorderen Loch (6d), einem dritten mittleren Loch (6e), einem vierten mittleren Loch (6f) und einem zweiten hinteren Loch (6h) versehen ist; und das zweite hintere Loch (6h), das dritte mittlere Loch (6e), das vierte mittlere Loch (6f) und das zweite vordere Loch (6d) der Reihe nach vertikal dem ersten hinteren Loch (5i), dem zweiten mittleren Loch (5h), dem ersten mittleren Loch (5g) beziehungsweise dem ersten vorderen Loch (5f) entspricht.

3. Integrierte Fußprothese mit Querbogen nach Anspruch 1, wobei die gekrümmte Sohlenplatte (A) eine Kohlenstofffaserverbundwerkstoffplatte ist und das Anpassungsstück (B) aus einer Aluminiumlegierung gefertigt ist.

## Revendications

1. Prothèse de pied intégrée avec arc transversal, comprenant une semelle incurvée (A), une pièce d'adaptation (B), un premier groupe de boulons (1), un deuxième groupe de boulons (2), un troisième groupe de boulons (3) et un quatrième groupe de boulons (4), dans laquelle la semelle incurvée (A) comprend une plaque plate arrière (5a), une plaque de transition arrière (5b), une plaque arquée centrale (5c), une plaque de transition centrale (5d) et une plaque plate avant (5e), la plaque plate arrière (5a), la plaque de transition arrière (5b), la plaque arquée centrale (5c), la plaque de transition centrale (5d) et la plaque plate avant s(5e) sont reliées uniformément et séquentiellement en une unité monobloc de l'arrière vers l'avant ; la pièce d'adaptation (B) comprend un tronc carré (6a), une plateforme (6b) et un corps principal (6c), le tronc carré (6a) est relié à demeure à une partie arrière du corps principal (6c) au moyen de la plateforme (6b) ; la plaque plate arrière (5a) et la plaque plate avant (5e) sont des lignes brisées, les lignes brisées sont formées en reliant deux lignes droites avec une différence de hauteur de 2 mm au moyen d'une transition arquée ; la plaque de transition arrière (5b) et la plaque de transition centrale (5d) passent chacune uniformément d'une forme d'arc convexe vers le haut à une forme droite, avec un rayon d'arc de 20 mm-25 mm, un angle central de 80° - 85° et une courbure allant de grande à petite ; la plaque arquée centrale (5c) présente une courbe transversale conçue en se référant à une courbe d'arc transversal de pied humain, et une extrémité avant et une extrémité arrière sont reliées au moyen d'une transition arquée douce ; la semelle incurvée (A) est entièrement définie par une courbe de semelle longitudinale (C), une courbe longitudinale externe (D) et une courbe longitudinale interne (E) dans une direction avant et arrière, la courbe de semelle longitudinale (C) comprend une première courbe longitudinale (5j), une deuxième courbe longitudinale (5k) et une troisième courbe longitudinale (51) ; la première courbe longitudinale (5j) est un arc concave vers le bas avec un rayon d'arc de 60 mm-70 mm et un angle central de 43° - 45°, la deuxième courbe longitudinale (5k) est un arc concave vers le haut avec un rayon d'arc de 50 mm - 60 mm et un angle central de 74° - 76°, et la première courbe longitudinale (5j) et la deuxième courbe longitudinale (5k) sont reliées séquentiellement et uniformément de manière tangentielle d'arrière en avant ; la troisième courbe longitudinale (51) est un arc concave vers le bas avec un rayon d'arc de 100 mm-110 mm et un angle central de 40° - 42°, et la deuxième courbe longitudinale (5k) et la troisième courbe longitudinale (51) sont reliées séquentiellement et uniformément de manière tangentielle d'arrière en avant ; des formes de la courbe longitudinale externe (D) et de la courbe longitudinale interne (E) décrivent une forme de base d'un arc longitudinal humain et présentent une courbure interne plus grande et une courbure externe plus petite ;
une expression mathématique de la courbe longitudinale externe (D) est : y = -0,0015x³+0,3313x²-3,5641x;
une expression mathématique de la courbe longitudinale interne (E) est : y = -0,0025x³+0,5492x²-9,6027x; et
la pièce d'adaptation (B) et la semelle incurvée (A) sont agencées verticalement, et sont reliées à demeure par le premier groupe de boulons (1), le deuxième groupe de boulons (2), le troisième groupe de boulons (3) et le quatrième groupe de boulons (4) ; une surface inférieure du corps principal (6c) de la pièce d'adaptation (B) présente la même taille que les surfaces arquées de la plaque de transition arrière (5b), de la plaque arquée centrale (5c) et de la plaque de transition centrale (5d) de la semelle incurvée (A) ; une partie centrale du corps principal (6c) est dotée de trois trous elliptiques d'un groupe de trous réducteurs de poids (6g), un axe court d'une ellipse est de 2 mm - 8 mm et un axe long de l'ellipse est de 9 mm - 30 mm ; et la surface inférieure du corps principal (6c) est une surface incurvée conçue selon la courbe d'arc transversal de pied humain, et s'adapte à une surface supérieure de la semelle incurvée (A).

2. Prothèse de pied intégrée avec arc transversal selon la revendication 1, dans laquelle la plaque de transition arrière (5b) de la semelle incurvée (A) est dotée d'un premier trou arrière (5i), la plaque arquée centrale (5c) est dotée d'un premier trou central (5g) et d'un deuxième trou central (5h), et la plaque de transition centrale (5d) est dotée d'un premier trou avant (5f) ; une partie inférieure du corps principal (6c) de la pièce d'adaptation (B) est doté d'un second trou avant (6d), d'un troisième trou central (6e), d'un quatrième trou central (6f) et d'un second trou arrière (6h) ; et le second trou arrière (6h), le troisième trou central (6e), le quatrième trou central (6f) et le second trou avant (6d) séquentiellement correspondent respectivement verticalement au premier trou arrière (5i), au deuxième trou central (5h), au premier trou central (5g) et au premier trou avant (5f).

3. Prothèse de pied intégrée avec arc transversal selon la revendication 1, dans laquelle la semelle incurvée (A) est une plaque composite en fibre de carbone et la pièce d'adaptation (B) est en alliage d'aluminium.
